# EUROPÄISCHE PATENTSCHRIFT

(11) **EP 1 363 868 B1**
(45) Veröffentlichungstag und Bekanntmachung des Hinweises auf die Patenterteilung: **01.11.2017**
(21) Anmeldenummer: 02701282.2
(22) Anmeldetag: 15.02.2002
(51) Int. Cl.: C07C 51/567, C07C 67/303, C07C 67/08, C08K 5/09, C08K 5/12

(54) **VERFAHREN ZUR HERSTELLUNG VON CYCLOHEXANDICARBONSÄUREN UND DEREN DERIVATEN**
METHOD FOR PRODUCING CYCLOHEXANE DICARBOXYLIC ACIDS AND THE DERIVATIVES THEREOF
PROCÉDÉ DE PRODUCTION D'ACIDES CYCLOHEXANEDICARBOXYLIQUES ET LEURS DÉRIVÉS

(30) Priorität: 16.02.2001 DE 10107366; 29.06.2001 DE 10131260; 12.12.2001 DE 10161010
(43) Veröffentlichungstag der Anmeldung: 26.11.2003
(73) Patentinhaber: BASF SE, 67056 Ludwigshafen am Rhein (DE)
(72) Erfinder: NOE, Ralf, 68163 Mannheim (DE); HIBST, Hartmut, 69198 Schriesheim (DE); HALBRITTER, Klaus, 69124 Heidelberg (DE); MAAS-BRUNNER, Melanie, 68165 Mannheim (DE); BREITSCHEIDEL, Boris, 67117 Limburgerhof (DE); KAIBEL, Gerd, 68623 Lampertheim (DE); MASSONNE, Klemens, 67098 Bad Dürkheim (DE); SALDEN, Axel, 70180 Stuttgart (DE); EBEL, Klaus, 68623 Lampertheim (DE); BERGNER, Eike, Johannes, 69198 Schriesheim (DE); HAESE, Frank, 24855 Bollingstedt (DE)
(86) Internationale Anmeldenummer: PCT/EP2002/001661
(87) Internationale Veröffentlichungsnummer: WO 2002/066412

(56) Entgegenhaltungen:
- EP-A- 0 603 825
- WO-A-98/33787
- DE-B- 1 263 296
- US-A- 2 794 811
- DATABASE CROSSFIRE BEILSTEIN [Online] Beilstein Institut zur Förderung der Chemischen Wissenschaften, Frankfurt am Main, DE; Database accession no. 36041 XP002197908 & BAILEY; HUDSON: JOURNAL OF THE AMERICAN CHEMICAL SOCIETY., Bd. 78, 1956, Seite 2806 AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 36618 XP002197909 & KOHLER, JANSEN: JOURNAL OF THE AMERICAN CHEMICAL SOCIETY., Bd. 60, 1938, Seiten 2142-2145, AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 808553 XP002197910 & AITKEN, R ALAN ET AL: TETRAHEDRON., Bd. 41, Nr. 7, 1985, Seiten 1329-1346, ELSEVIER SCIENCE PUBLISHERS, AMSTERDAM., NL ISSN: 0040-4020
- DATABASE CROSSFIRE BEILSTEIN [Online] Beilstein Institut zur Förderung der Chemischen Wissenschaften, Frankfurt am Main, DE; Database accession no. 808572 XP002197911 & RICE ET AL: JOURNAL OF THE AMERICAN CHEMICAL SOCIETY., Bd. 75, 1953, Seiten 4911-4912, AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 4076855 XP002197912 & ITO, YOSHIO N ET AL: HELVETICA CHIMICA ACTA., Bd. 77, Nr. 8, 1994, Seiten 2071-2110, VERLAG HELVETICA CHIMICA ACTA. BASEL., CH ISSN: 0018-019X
- DATABASE CROSSFIRE BEILSTEIN [Online] Beilstein Institut zur Förderung der Chemischen Wissenschaften, Frankfurt am Main, DE; Database accession no. 2199738 XP002197913 & ZOLLER, T ET AL: TETRAHEDRON LETTERS., Bd. 38, Nr. 19, 1997, Seiten 3409-3412, ELSEVIER SCIENCE PUBLISHERS, AMSTERDAM., NL ISSN: 0040-4039
- DATABASE CROSSFIRE BEILSTEIN [Online] Beilstein Institut zur Förderung der Chemischen Wissenschaften, Frankfurt am Main, DE; Database accession no. 808616 XP002197914 & NASAROW, KUTSCHEROW: AKADEMIYA NAUK SSSR IZVESTIYA. SERIYA KHIMICHESKAYA, 1954, Seiten 329-333, MOSCOW, RU
- DATABASE CROSSFIRE BEILSTEIN [Online] Beilstein Institut zur Förderung der Chemischen Wissenschaften, Frankfurt am Main, DE; Database accession no. 808613 XP002197915 & FIESER, NOVELLO: JOURNAL OF THE AMERICAN CHEMICAL SOCIETY., Bd. 64, 1942, Seiten 802-807, AMERICAN CHEMICAL SOCIETY, WASHINGTON, DC., US ISSN: 0002-7863
- DATABASE CROSSFIRE BEILSTEIN [Online] Beilstein Institut zur Förderung der Chemischen Wissenschaften, Frankfurt am Main, DE; Database accession no. 1415615 XP002197916 & RUSSELL, G A ET AL: JOURNAL OF THE AMERICAN CHEMICAL SOCIETY., Bd. 94, 1972, Seiten 1693-1698, AMERICAN CHEMICAL SOCIETY, WASHINGTON, DC., US ISSN: 0002-7863

## Beschreibung

Die Erfindung betrifft ein Verfahren zur Herstellung von Cyclohexandicarbonsäuren oder deren Mono- oder Diestern, umfassend die Umsetzung von Dien-Maleinsäureanhydrid-Gemischen, insbesondere von Butadien-Maleinsäureanhydrid-Gemischen oder von Gemischen aus Maleinsäureanhydrid und mindestens einem C5-Dien, zu alkylsubstituierten oder unsubstituierten Cyclohexendicarbonsäureanhydriden in kondensierter Phase.

Darüber hinaus betrifft die Erfindung auch die erfindungsgemäß hergestellten alkylsubstituierten oder unsubstituierten Cyclohexandicarbonsäuren oder deren Mono- oder Diester, Gemische enthaltend die erfindungsgemäß hergestellten alkylsubstituierten oder unsubstitutierten Cyclohexandicarbonsäuren oder deren Mono- oder Diester sowie die Verwendung der erfindungsgemäß hergestellten Gemische als Weichmacher in Kunststoffen, wobei der Kunststoff Polyvinylchlorid (PVC) oder Polyvinylbutyral (PVB) ist.

Bislang wurden als Weichmacher in Kunststoffen, wie z.B. PVC, sehr häufig Phthalsäureester, wie z.B. Dibutyl-, Dioctyl- oder Diisononylester der Phthalsäure, verwendet, wie dies z.B. aus der FR-A 23 97 131 hervorgeht. Diesen wird jedoch seit kurzer Zeit nachgesagt, dass sie gesundheitlich nicht unbedenklich sind, so dass ihre Verwendung in Kunststoffen zur Herstellung von z.B. Kinderspielzeug immer stärker in der Kritik steht und in einigen Ländern bereits verboten ist. Im Tierversuch wurde mittlerweile gezeigt, dass Phthalate zu einer Peroxisomenproliferation führen können, welche in ursächlichem Zusammenhang mit den bei Maus und Ratte in Langzeitstudien aufgetretenen Lebertumoren steht.

Die Verwendung von einigen Cyclohexan-1,2-dicarbonsäureestern als Weichmacher ist ebenfalls aus dem Stand der Technik bekannt. So ist die Verwendung von Cyclohexandicarbonsäuredimethyl oder -diethylestem (DE-A 28 23 165) und Cyclohexan-1,2-dicarbonsäuredi(2-ethylhexyl)ester (DE-A 12 63 296) als Weichmacher in Kunststoffen beschrieben.

In PCT/EP 98/08346 wird offenbart, dass Cyclohexanpolycarbonsäuren und Derivate davon als Weichmacher verwendet werden können. In diesem Zusammenhang wird offenbart, dass Cyclohexanpolycarbonsäuren und Derivate davon im Vergleich mit den bislang hauptsächlich als Weichmacher verwendeten Phthalaten eine niedrigere Dichte und Viskosität aufweisen und darüber hinaus u.a. auch zu einer Verbesserung der Kälteflexibilität des Kunststoffs gegenüber der Verwendung der entsprechenden Phthalate als Weichmacher führen. Des Weiteren wird in PCT/EP 98/08346 offenbart, dass Cyclohexanpolycarbonsäuren und Derivate davon ein besseres Verarbeitungsverhalten im Dry-Blend und als Folge eine erhöhte Produktionsgeschwindigkeit sowie in Plastisol-Verarbeitungen Vorteile durch eine deutlich niedrigere Viskosität gegenüber den entsprechenden Phthalaten aufweisen.

Die EP-A 0 603 825 betrifft ein Verfahren zur Herstellung von 1,4-Cyclohexandicarbonsäure durch Hydrierung von Terephthalsäure unter Verwendung eines geträgerten Palladium-Katalysators, wobei als Träger Aluminiumoxid, Siliciumdioxid oder Aktivkohle verwendet wird.

In der DE-A 199 77 977.2 wird die Verwendung einer Cyclohexanpolycarbonsäure oder eines Derivats davon, welche(s) keine biologisch signifikante Peroxisomenproliferation bedingt, d. h. als toxikologisch unbedenklich einzustufen ist, als Weichmacher für Kunststoffe offenbart.

Die DE-A 199 27 978.0 betrifft ausgewählte Cyclohexan-1,3- und -1,4-dicarbonsäureester, die mittels Hydrierung der entsprechenden Isophthalsäure- und Terephthalsäureester durch Inkontaktbringen mit einem Wasserstoff enthaltenden Gas in Gegenwart eines Katalysators, der als Aktivmetall mindestens ein Metall der VIII. Nebengruppe des Periodensystems alleine oder zusammen mit mindestens einem Metall der I. oder VII. Nebengruppe des Periodensystems, aufgebracht auf einen Träger, hergestellt werden. Die Verwendung als Weichmacher wird ebenfalls erwähnt.

Prinzipiell sind ausgewählte Diester von Norbonan-2,3-dicarbonsäure, 4-Methylcyclohexan-1,2-dicarbonsäure und 3-Methylcyclohexan-1,2-dicarbonsäure bekannt, beispielsweise aus der JP 2000-034492, der JP 70-11074, der JP 71-73342, der JP 52-95025 oder der JP 71-38205. Methylcyclohexan-1,2-dicarbonsäureester auf Basis von Alkoholen mit 6 bis 28 C-Atomen sind bislang nur als Weichmacher für Polyolefine beschrieben, beispielsweise in der JP 63-06252. Als Weichmacher für PVC sind lediglich Ester der Norbonan-2,3-dicarbonsäure mit n-Octanol oder 2-Ethylhexanol als Alkoholkomponente beschrieben (S. Matsuda, S. Kikkawa, Kogyo Kagaku Zasshi (1959), 62,1838-1841).

In der Schrift US 2,794,811 wird ein Verfahren zur Herstellung von cis-Cyclohexan-1,2-dicarbonsäureanhydriden offenbart. Demgemäß können die Cyclohexanderivate durch Hydrierung eines Cyclohexenderivats erhalten werden, das mittels Diels-Alder-Reaktion aus einem Dien und Maleinsäureanhydrid erhalten wird. Die Veresterung der Anhydride wird in dieser Schrift nicht beschrieben.

Die Verwendung toxikologisch unbedenklicher Weichmacher ist insbesondere für solche Kunststoffe wichtig, die zur Herstellung von Gegenständen des täglichen Gebrauchs eingesetzt werden. Insbesondere Polyvinylchlorid wird zur Herstellung vieler Alltagsgegenstände und auch Kinderspielzeug eingesetzt.

Die bisher genutzten Verfahren zur Herstellung der Cyclohexancarbonsäurederivate basieren alle auf der Hydrierung der zugrundeliegenden Phthalsäureester. Die Herstellung der Weichmacher wird dabei um eine Reaktionsstufe verlängert und das Produkt verteuert.

Der vorliegenden Erfindung lag daher die primäre Aufgabe zugrunde, ein Verfahren zur Herstellung von alkylsubstituierten oder unsubstituierten Cyclohexandicarbonsäuren, deren Mono- oder Diester bereitzustellen, bei dem kostengünstige Rohstoffe eingesetzt werden, die einfach und in großen Mengen zugänglich sind.

Erfindungsgemäß wird diese Aufgabe gelöst durch ein Verfahren zur Herstellung einer Cyclohexandicarbonsäure, deren Mono- oder Diester umfassend die folgenden Abfolgen der Schritte (1) bis (3):
(1) Umsetzung eines Butadien-Maleinsäureanhydrid-Gemischs zu Cyclohexendicarbonsäureanhydrid in kondensierter Phase;
(2) Esterbildung aus einem Cyclohexendicarbonsäureanhydrid;
(3) Hydrierung des Cyclohexenderivats aus Schritt (2) zu dem entsprechenden Derivat von Cyclohexan;
oder
(1) Umsetzung eines Butadien-Maleinsäureanhydrid-Gemischs zu Cyclohexendicarbonsäureanhydrid in kondensierter Phase;
(3) Hydrierung des alkylsubstituierten oder unsubstituierten Cyclohexendicarbonsäureanhydrid zu Cyclohexandicarbonsäureanhydrid;
(2) Esterbildung aus dem Cyclohexandicarbonsäureanhydrid, wobei das Butadien-Maleinsäureanhydrid-Gemisch für die Umsetzung gemäß Schritt (1) durch ein Verfahren erhalten wird umfassend die Oxidation von Strömen enthaltend n-Buten oder Butadien oder ein Gemisch davon oder durch ein Verfahren umfassend die Oxidation von n-Buten zu Butadien oder durch ein Verfahren umfassend die Oxidation von n-Buten zu einem Butadien-Maleinsäureanhydrid-Gemisch
   Die Cyclohexandicarbonsäuren, deren Mono- oder Diester, können alkylsubstituiert oder unsubstituiert sein..

Dabei wird im Rahmen der vorliegenden Erfindung unter einer alkylsubstituierten Cyclohexandicarbonsäure ein Cyclohexanderivat mit einem Alkylsubstituenten am aliphatischen Ring verstanden oder auch ein mit einem Alkylrest überbrücktes Cyclohexanderivat, d.h. eine bicyclische Struktur.

Im Rahmen der vorliegenden Erfindung ist es bevorzugt, wenn die Cyclohexandicarbonsäure unsubstituiert ist oder einen Methylsubstituenten aufweist. Erfindungsgemäß kann es sich bei dem Methylsubstituenten auch um eine Methylenbrücke handeln.

Die erfindungsgemäße Aufgabe wird auch gelöst durch ein Verfahren zur Herstellung einer alkylsubstituierten Cyclohexandicarbonsäure, deren Mono- oder Diester umfassend die folgenden Abfolgen der Schritte (1) bis (3):
(1) Umsetzung eines Gemischs aus Maleinsäureanhydrid und mindestens ein C5-Dien zu einem alkylsubstituierten Cyclohexendicarbonsäureanhydrid in kondensierter Phase;
(2) Esterbildung aus einem alkylsubstituierten Cyclohexendicarbonsäureanhydrid;
(3) Hydrierung des alkylsubstituierten Cyclohexenderivats aus Schritt (2) zu dem entsprechenden Derivat von Cyclohexan;
oder
(1) Umsetzung eines Gemischs aus Maleinsäureanhydrid und mindestens ein C5-Dien zu einem alkylsubstituierten Cyclohexendicarbonsäureanhydrid in kondensierter Phase;
(3) Hydrierung des alkylsubstituierten Cyclohexendicarbonsäureanhydrid zu Cyclohexandicarbonsäureanhydrid;
(2) Esterbildung aus dem alkylsubstituierten Cyclohexandicarbonsäureanhydrid.

Als Diene werden Butadien oder Diene mit 5 Kohlenstoffatomen eingesetzt. Erfindungsgemäß geeignete Diene sind Diene mit konjugierten Doppelbindungen. Diene mit isolierten Doppelbindungen reagieren unter den erfindungsgemäßen Reaktionsbedingungen nicht.

Die vorliegende Erfindung betrifft daher ein Verfahren zur Herstellung einer Cyclohexandicarbonsäure , deren Mono- oder Diester, umfassend die folgenden Abfolgen der Schritte (1) bis (3):
(1) Umsetzung eines Butadien-Maleinsäureanhydrid-Gemischs zu Cyclohexendicarbonsäureanhydrid in kondensierter Phase;
(2) Esterbildung aus einem Cyclohexendicarbonsäureanhydrid;
(3) Hydrierung des Cyclohexenderivats aus Schritt (2) zu dem entsprechenden Derivat von Cyclohexan;
oder
(1) Umsetzung eines Butadien-Maleinsäureanhydrid-Gemischs zu Cyclohexendicarbonsäureanhydrid in kondensierter Phase;
(3) Hydrierung des Cyclohexendicarbonsäureanhydrid zu Cyclohexandicarbonsäureanhydrid;
(2) Esterbildung aus dem Cyclohexandicarbonsäureanhydrid,
   wobei das Butadien-Maleinsäureanhydrid-Gemisch für die Umsetzung gemäß Schritt (1) durch ein Verfahren erhalten wird umfassend die Oxidation von Strömen enthaltend n-Buten oder Butadien oder ein Gemisch davon oder durch ein Verfahren umfassend die Oxidation von n-Buten zu Butadien oder durch ein Verfahren umfassend
   die Oxidation von n-Buten zu einem Butadien-Maleinsäureanhydrid-Gemisch.

Der Vorteil dieses Verfahrens liegt in der Verwendung einer Maleinsäureanhydrid-Rohlösung und eines Roh-Butadien-Gemisches für die Umsetzung gemäß Schritt (1), in denen Nebenprodukte und inerte Bestandteile wie beispielsweise Stickstoff, Sauerstoff oder n- oder iso-Butane vorliegen, und damit der Einsparung eines Aufarbeitungsschrittes zur Reingewinnung von Butadien und Maleinsäureanhydrid. In einer weiteren bevorzugten Ausführungsform betrifft die vorliegende Erfindung ein Verfahren zur Herstellung einer alkylsubstituierten Cyclohexandicarbonsäure, deren Mono- oder Diester, umfassend die folgenden Abfolgen der Schritte (1) bis (3):
(1) Umsetzung eines Gemischs aus Maleinsäureanhydrid und mindestens ein C5-Dien zu einem alkylsubstituierten Cyclohexendicarbonsäureanhydrid in kondensierter Phase;
(2) Esterbildung aus einem alkylsubstituierten Cyclohexendicarbonsäureanhydrid;
(3) Hydrierung des alkylsubstituierten Cyclohexenderivats aus Schritt (2) zu dem entsprechenden Derivat von Cyclohexan;
oder
(1) Umsetzung eines Gemischs aus Maleinsäureanhydrid und mindestens ein C5-Dien zu einem alkylsubstituierten Cyclohexendicarbonsäureanhydrid in kondensierter Phase;
(3) Hydrierung des alkylsubstituierten Cyclohexendicarbonsäureanhydrid zu Cyclohexandicarbonsäureanhydrid;
(2) Esterbildung aus dem alkylsubstituierten Cyclohexandicarbonsäureanhydrid.

Erfindungsgemäß wird der Begriff "Cyclohexandicarbonsäuren, deren Mono- oder Diester," verwendet. Die eingesetzten Ester sind Alkyl-, Cycloalkyl- sowie Alkoxyalkylester, wobei die Alkyl-, Cycloalkyl- sowie Alkoxyalkylgruppen in der Regel 1 bis 30, vorzugsweise 2 bis 20 und besonders bevorzugt 3 bis 18 Kohlenstoffatome umfassen und verzweigt oder linear sein können.

Als Roh-Butadien-Gemische eignen sich Butadien-haltige Gemische, welche überwiegend aus Kohlenwasserstoffen bestehen, wobei die von Butadien verschiedenen Kohlenwasserstoffe unter den Bedingungen des Verfahrensschrittes (1) inert sind.

Solche inerten Kohlenwasserstoffe sind beispielsweise Alkane, Monoalkene, Cycloalkane, Benzol und Dialkylbenzole wie Propan, n-Butan, 2-Methylpropan, 1-Buten, cis-2-Buten, trans-2-Buten, iso-Buten, Cyclohexan, Benzol, Toluol und die Xylole.

Für den Anteil an Butadien in diesen Roh-Butadien-Gemischen gibt es im Allgemeinen nach unten hin keine Begrenzung, jedoch ist in der Regel im Hinblick etwa auf die Raum-Zeit-Ausbeute des Verfahrensschrittes (1) ein möglichst hoher Gehalt an Butadien in solchen Gemischen wünschenswert. Auch kann sich der Gehalt an Butadien im Roh-Butadien-Gemisch danach richten, welche Zusammensetzung der Kohlenwasserstoffstrom haben soll, welcher nach der Verfahrensstufe (1) zurückbleibt: Soll der Kohlenwasserstoffstrom beispielsweise nach der Umsetzung gemäß Verfahrensschritt (1) praktisch frei sein von Butadien, so ist der Anteil an Butadien im eingesetzten Gemisch entsprechend einzustellen.

Vorzugsweise enthalten derartige Roh-Butadien-Gemische 20 bis 95 und insbesondere 40 bis 50 Gew.-% Butadien.

Ein besonders kostengünstiges Roh-Butadien-Gemisch ist der sogenannte Roh-C₄-Schnitt, dessen Butadien-Gehalt in der Regel 40 bis 50 Gew.-% beträgt und der in großen Mengen in der Industrie anfällt (vgl. etwa K. Weissermel, H.-J. Arpe, Industrielle Organische Chemie, VCH Weinheim, 5. Auflage, 1998 sowie FR-A 1 343 169, DE-A 14 43 362 und DE-A 14 68 843). Mit dem Roh-C₄-Schnitt lässt sich der Verfahrensschritt (1) so durchführen, dass ein praktisch Butadienfreier Kohlenwasserstoffstrom abfällt. Dieser Kohlenwasserstoffstrom entspricht hinsichtlich seiner Zusammensetzung dem sogenannten Raffinat I, welches ansonsten normalerweise durch Butadien-Extraktion aus dem C₄-Schnitt aus Crakkern erhalten wird, und er kann daher als Ersatz für Raffinat I dienen.

Eine Abtrennung von Butadien aus C₄-Schnitten ist allgemein bekanntermaßen wegen der Bildung von Azeotropen mit anderen Bestandteilen des C₄-Schnittes destillativ in der Regel nicht möglich, so dass üblicherweise eine aufwendige Extraktion des Butadiens aus solchen C₄-Schnitten durchgeführt werden muss.

Daher stellt die Verfahrensstufe (1) für sich allein auch ein neues Verfahren zur Entfernung von Butadien aus Butadien-haltigen Kohlenwasserstoff-Gemischen und insbesondere aus C₄-Schnitten dar (vgl. etwa K. Weissermel, H.-J. Arpe, Industrielle Organische Chemie, VCH Weinheim, 5. Auflage, 1998).

Es handelt sich also beim Verfahrensschritt (1) der vorliegenden Erfindung auch um ein Verfahren zur Abtrennung von Butadien aus Butadien-haltigen Kohlenwasserstoffströmen, wobei der Kohlenwasserstoffstrom mit Maleinsäureanhydrid umgesetzt wird und das dabei gebildete Cyclohexendicarbonsäureanhydrid vom verbleibenden Kohlenwasserstoffstrom abgetrennt wird.

Das Butadien-Maleinsäureanhydrid-Gemisch für die Umsetzung gemäß Schritt (1) wird durch ein Verfahren erhalten, umfassend die Oxidation von Strömen enthaltend n-Buten oder Butadien oder ein Gemisch davon.

Im Rahmen der vorliegenden Erfindung wird als Butadien bevorzugt 1,3-Butadien eingesetzt. Erfindungsgemäß kann das Butadien-Maleinsäureanhydrid-Gemisch für die Umsetzung gemäß Schritt (1) jedoch auch durch ein Verfahren erhalten werden, umfassend die Oxidation von Strömen enthaltend n-Butan.

Im Rahmen der vorliegenden Erfindung wird unter Strömen enthaltend n-Buten oder Butadien oder ein Gemisch davon bevorzugt eine C4-Fraktion eines Raffinat-Stroms verstanden. Dabei handelt es sich beispielsweise um Gemische mit folgender Zusammensetzung: Butan 10 bis 90 Gew.-%, Buten 10 bis 90 Gew.-%, wobei die Buten-Fraktion folgende Zusammensetzung haben kann: 0 bis 100 Gew.-%, insbesondere 0 bis 80 Gew.-%, besonders bevorzugt 0 bis 50 Gew.-% Buten-1, 0 bis 100 Gew.-%, insbesondere 0 bis 80 Gew.-%, besonders bevorzugt 0 bis 50 Gew.-% cis-Buten-2, 0 bis 100 Gew.-%, insbesondere 0 bis 80 Gew.-%, besonders bevorzugt 0 bis 50 Gew.-% trans-Buten-2, 0 bis 10 Gew.-% iso-Buten, wobei es möglich ist, dass andere Kohlenwasserstoffe, insbesondere C5-Kohlenwasserstoffe, in geringen Mengen, beispielsweise 0 bis 20 Gew.-%, insbesondere 0 bis 10 Gew.-%, im Strom vorliegen. Darüber hinaus können auch die reinen n-Butene eingesetzt werden oder ein Gemisch aus einem reinen n-Buten und Butadien.

Als besonders bevorzugter Einsatzstoff wird das sogenannte Raffinat II verwendet, also ein n-Buten-haltiges C4-Kohlenwasserstoffgemisch, wie es aus dem C4-Schnitt von Crackern nach Abtrennung der Hauptmenge an iso-Buten erhalten wird. Für das erfindungsgemäße Verfahren ist es jedoch nicht zwingend notwendig, dass das darin auch enthaltene Butadien entfernt wird. Damit entfällt ein weiterer Reinigungsschritt.

Im Rahmen der vorliegenden Erfindung werden insbesondere n-Buten-reiche Ströme eingesetzt, die mindestens 60 Gew.-%, insbesondere mindestens 80 Gew.-% n-Buten enthalten und in kleineren Mengen n- und iso-Butan sowie weitere Kohlenwasserstoffe.

Die Oxidation von Strömen enthaltend n-Buten oder Butadien oder ein Gemisch davon lässt sich über die Verwendung verschiedener Katalysatoren und die Wahl der Reaktionsbedingungen weitgehend steuern.

Erfindungsgemäß kann das Butadien-Maleinsäureanhydrid-Gemisch für die Umsetzung gemäß Schritt (1) beispielsweise erhalten werden durch ein Verfahren umfassend die Oxidation von n-Buten zu Butadien. Es ist erfindungsgemäß möglich, dass im Anschluss an diese Oxidation dem Roh-Butadien Maleinsäureanhydrid (MSA) zugesetzt wird, um ein Butadien-Maleinsäureanhydrid-Gemisch für die Umsetzung gemäß Schritt (1) zu erhalten.

In einer bevorzugten Ausführungsform betrifft die vorliegende Erfindung ein Verfahren zur Herstellung einer Cyclohexandicarbonsäure, deren Mono- oder Diester, das nach folgendem Schema (I) abläuft: 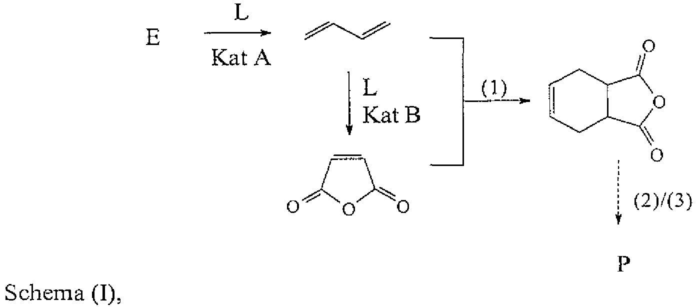 wobei in Schema (I) E für n-Butene (Raffinat), L für Luft, Kat A für Katalysatorsystem A, Kat B für Katalysatorsystem B und P für Cyclohexan-1,2-dicarbonsäureester steht.

Dabei sind die Schritte (1) bis (3) des erfindungsgemäßen Verfahrens im Schema wie auch in den folgenden Schemen gekennzeichnet.

Die Umsetzung von Butenen an einem Katalysatorsystem A (Kat A), beispielsweise einem Katalysator der Zusammensetzung (Fe, Co)-Mo-O + Bi-W-O, zu Butadien kann mit hohen Butadienausbeuten von 85 bis 90 % durchgeführt werden. In der US 4,595,788 und der US 4,547,615 werden Verfahren zur Herstellung von Butadien bzw. konjugierten Diolefinen beschrieben. Durch Umsetzung von C4-Gemischen enthaltend n-Buten wird mit Katalysatoren auf Basis von Molybdän, Bismut oder Nickel Butadien in Ausbeuten von über 80 % erhalten. Wie Vergleichsversuche gezeigt haben, entsteht bei derartigen Umsetzungen neben Butadien auch in geringen Mengen Maleinsäureanhydrid (1 bis 10%).

Da das Butadien für die Umsetzung gemäß Schritt (1) eingesetzt wird, ist ein MSA-Anteil im Butadien nicht störend. Damit kann die Dehydrierung zur Gewinnung von Butadien als Rohstoff für die nachfolgende Umsetzung besonders wirtschaftlich bei hohem Butenumsatz unter Einbeziehung einer Zusatzausbeute an MSA betrieben werden, beispielsweise nach einem Verfahren gemäß Schema (III).

In einer bevorzugten Ausführungsform der Erfindung wird das n-Buten zur Herstellung des Butadien-Maleinsäureanhydrid-Gemischs durch ein Verfahren erhalten, umfassend die Oxidation von Strömen enthaltend n-Butan.

Ausgehend von linearen Butenen und Buten-haltigen Einsatzstoffen ist es ebenfalls möglich, Mischungen aus Butadien und MSA in geeigneten Mischungsverhältnissen zu erhalten.

Daher betrifft die Erfindung auch ein Verfahren zur Herstellung einer Cyclohexandicarbonsäure, deren Mono- oder Diester, bei dem das Butadien-Maleinsäureanhydrid-Gemisch für die Umsetzung gemäß Schritt (1) durch ein Verfahren erhalten wird, umfassend die Oxidation von n-Buten zu einem Butadien-Maleinsäureanhydrid-Gemisch.

In einer weiteren bevorzugten Ausführungsform betrifft die Erfindung ein Verfahren zur Herstellung einer Cyclohexandicarbonsäure, deren Mono- oder Diester, das nach folgendem Schema (II) abläuft: 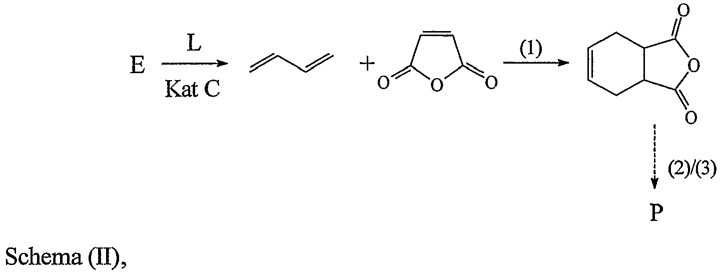 wobei in Schema (II) E für n-Butene (Raffinat), L für Luft, Kat C für Katalysatorsystem C und P für Cyclohexan-1,2-dicarbonsäureester steht.

Katalysatorsystem C (Kat C) ist dabei ein Katalysatorsystem, das geeignet ist, um die Reaktion von n-Buten zu einem Butadien-Maleinsäureanhydrid-Gemisch zu katalysieren.

Die Rohmischung aus MSA und Butadien kann anschließend in einem als Absorptionsmedium für das MSA verwendeten, hochsiedenden, inerten Lösungsmittel gemäß Schritt (1) umgesetzt werden.

Bei gemäßigten Reaktionsbedingungen kann man durch die Oxidation ungesättigter n-C4-Kohlenwasserstoffe an verschiedenen Heterogenkontakten Mischungen von Maleinsäureanhydrid (MSA) und dem Zwischenprodukt Butadien erhalten. Durch eine geeignete Abstimmung von Reaktionsbedingungen und Katalysator lassen sich sowohl Butadien als auch MSA erhalten. Geeignete Bedingungen und Katalysatoren sind beispielsweise beschrieben in K. Weissermel, H.-J. Arpe, "Industrielle Organische Chemie", 4. Auflage, 1994, VCH Weinheim.

Die weitere Umsetzung von Butadien zu Maleinsäureanhydrid gelingt an einem zweiten Katalysatorsystem B (Kat B), beispielsweise SbMo_{3,06}Ti_{0,6}Nb_{0,1}Sn_{0,8}Oₓ/TiO₂. Im Feed enthaltenes Maleinsäureanhydrid stört nicht und bleibt im Produktstrom unzersetzt enthalten. Bei einer stufenweisen Umsetzung von Butenen an einem ersten Katalysatorsystem A zu Butadien und anschließender weiterer Umsetzung an einem zweiten Katalysatorsystem B können dementsprechend hohe Gesamtausbeuten erhalten werden, so dass das Verhältnis von Butadien und MSA im Produktstrom einstellbar ist.

Beispielsweise in der DE 28 13 424 werden geeignete Bedingungen für die Oxidation von Butadien zu Maleinsäureanhydrid mit Katalysatoren enthaltend Oxide von Antimon und Molybdän beschrieben.

Aus dem Reaktoreffluent wird MSA durch Absorption in einem hochsiedenden inerten Lösungsmittel als Rohlösung gewonnen. Die Absorptionstemperatur liegt bevorzugt oberhalb 55 °C, um die Kristallisation von MSA (Smp. 55 °C) zu vermeiden. Leichter siedende Komponenten (Butadien, Nebenprodukte, Verbrennungswasser, Kohlendioxide) werden kaum absorbiert und können aus dem Abgas beispielsweise durch Pressure-Swing-Absorption/Temperature-Swing-Absorption (PSA/TSA) leicht zurückgewonnen werden.

Im Unterschied zu technischen Prozessen zur MSA-Herstellung aus C4-Kohlenwasserstoffen wird dieser Strom anschließend aber nicht als Feed der Gasphasenoxidation zur Erhöhung der MSA-Ausbeute verwendet. Vielmehr ist das Rohgemisch der Leichtsieder, das überwiegend Butadien sowie weitere Olefine und Butane enthält, im Rahmen der vorliegenden Erfindung ein geeigneter Rohstoff für die Umsetzung gemäß Schritt (1) im Sinne einer Diels-Alder-Reaktion mit der Roh-MSA-Lösung. Es kann mit MSA in dem inerten Lösungsmittel, das zur Absorption von MSA verwendet wird, zur Reaktion gebracht werden. Als Diels-Alder Produkt erhält man Cyclohexendicarbonsäureanhydrid. Geeignete Bedingungen zur Durchführung der Umsetzung gemäß Schritt (1) sind beispielsweise in "Organic Syntheses", Coll. Vol. IV, 1963, J. Wiley&Sons, New York, beschrieben.

Eine weitere Ausführungsform des erfindungsgemäßen Verfahrens beinhaltet die oxidative Dehydrierung eines n-Buten enthaltenden Gemisches zu einem Strom, der im Wesentlichen aus Butadien und einer Nebenausbeute an MSA besteht. Ein Teilstrom, im entsprechend der für die Umsetzung gemäß Schritt (1) gewünschten Verhältnis, des Butadien und wenig MSA enthaltenden Gemischs wird in einer zweiten Oxidation zu MSA umgesetzt. Der nicht weiter umgesetzte Teilstrom der ersten Umsetzung und der Produktstrom der zweiten Oxidation werden in kondensierter Phase gemäß Schritt (1) zum Cyclohexendicarbonsäureanhydrid umgesetzt. Der Reaktionsaustrag aus der ersten Oxidation kann gasförmig in die zweite Oxidation eingesetzt und dort weiteroxidiert werden. Im Anschluss an die zweite Oxidation wird MSA dann aus dem Reaktoreffluent durch Absorption in einem hochsiedenden inerten Lösungsmittel als Rohlösung gewonnen werden. In die Rohlösung von MSA im hochsiedenden Lösungsmittel kann der Teilstrom, der das nicht weiter umgesetzte gasförmige Butadien aus der ersten Oxidation enthält, eingetragen werden. Die Umsetzung gemäß Schritt (1) kann direkt mit dem so erhaltenen Gemisch der Roh-Produkte durchgeführt. werden.

Insbesondere betrifft die Erfindung in einer weiteren Ausführungsform ein Verfahren zur Herstellung einer Cyclohexandicarbonsäure oder eines Derivats davon, das nach folgendem Schema (III) abläuft: 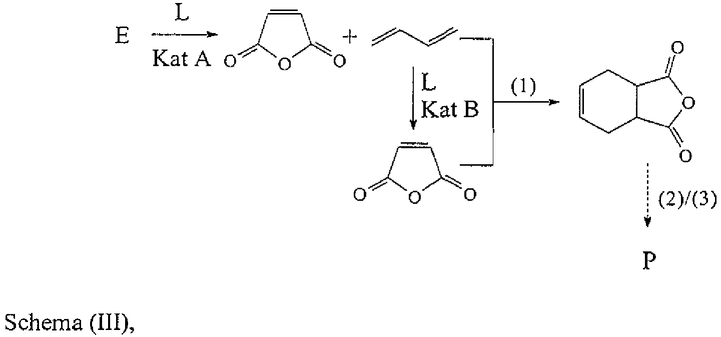 wobei in Schema (III) E für n-Butene (Raffinat), L für Luft, Kat A für Katalysatorsystem A, Kat B für Katalysatorsystem B und P für Cyclohexan-1,2-dicarbonsäureester steht.

In einer weiteren Ausführungsform der vorliegenden Erfindung wird das Butadien-Maleinsäureanhydrid-Gemisch vor der Umsetzung gemäß Schritt (1) einer Behandlung unterzogen. Bei einer Behandlung im Sinne der vorliegenden Erfindung kann es sich beispielsweise um das Abtrennen eines Nebenprodukts oder insbesondere um das Anreichern mit einer Komponente handeln, um eine für die Umsetzung gemäß Schritt (1) geeignetes stöchiometrisches Verhältnis der Komponenten zu erhalten.

Es ist erfindungsgemäß jedoch ebenso möglich, dass das Butadien-Maleinsäureanhydrid-Gemisch vor der Umsetzung gemäß Schritt (1) keiner Behandlung unterzogen wird.

Erfindungsgemäß ist es außerdem möglich, dass das Butadien-Maleinsäureanhydrid-Gemisch für die Umsetzung gemäß Schritt (1) aus Roh-Butadien und Roh-Maleinsäureanhydrid erhalten wird. Unter Roh-Butadien und Roh-Maleinsäureanhydrid werden dabei im Rahmen der vorliegenden Erfindung bevorzugt Ströme verstanden, die mindestens 60 Gew.-%, insbesondere mindestens 80 Gew.-% Butadien bzw. Maleinsäureanhydrid enthalten.

Durch die erfindungsgemäße in-situ Herstellung von Butadien und direkte Umsetzung gemäß Schritt (1) kann die aufwendige Verwendung von Depotverbindungen, welche im homogenen Reaktionsmedium der Reaktion die Dienen freisetzen, wie beispielsweise in der DE 1 082 908 beschrieben, vermieden werden. Die Verwendung von Depotverbindungen ist in dem dort beschriebenen Fall nötig, um die Verwendung von polymerisationsfreudigen Dienen in reiner Form zu vermeiden.

Im Rahmen der vorliegenden Erfindung werden bevorzugt als Diene neben Butadien auch C5-Diene, das heißt Diene mit 5 C-Atomen, eingesetzt. Neben den reinen C5-Dienen können besonders vorteilhaft auch Gemische verschiedener Diene eingesetzt werden. Erfindungsgemäß wird beispielsweise ein C5-Schnitt in die Diels-Alder Reaktion gemäß Schritt (1) eingesetzt. Bei dem C5-Schnitt handelt es sich um ein Gemisch verschiedener C5-Kohlenwasserstoffe, das beispielsweise beim Cracken längerkettiger Kohlenwasserstoffe anfällt. Der Vorteil bei der Verwendung des C5-Schnitts liegt darin, dass eine aufwendige und teure Reinigung der einzelnen C5-Diene vermieden wird. Daher betrifft die vorliegende Erfindung in einer bevorzugten Ausführungsform ein Verfahren zur Herstellung einer alkylsubstituierten Cyclohexandicarbonsäure oder eines Derivats davon, wobei als C5-Diene C5-Schnitte aus einem Crack-Verfahren eingesetzt werden.

Von den C5-Kohlenwasserstoffen des C5-Schnitts reagieren in der Diels-Alder-Reaktion mit Maleinsäureanhydrid (MSA) gemäß Schritt (1) nur die Diene Cyclopentadien, Isopren und Piperylen, das heißt die konjugierten Diene. Beispielsweise sind im C5-Schnitt aus Crackern 5 bis 30 Gew.-%, bevorzugt 10 bis 25 Gew.-%, insbesondere 15 bis 20 Gew.-% Cyclopentadien, das teilweise als Dicyclopentadien vorliegen kann, enthalten, vorzugsweise 17 Gew.-%; 5 bis 25 Gew.-%, bevorzugt 10 bis 20 Gew.-%, insbesondere 12 bis 18 Gew.-% Isopren, vorzugsweise 15 Gew.-%; und etwa 5 bis 25 Gew.-%, bevorzugt 10 bis 20 Gew.-%, insbesondere 8 bis 12 Gew.-% Piperylen, vorzugsweise 10 Gew.-%. Darüber hinaus kann der C5-Schnitt noch andere Verbindungen enthalten, die unter den erfindungsgemäßen Reaktionsbedingungen inert sind. Der C5-Schnitt kann beispielsweise auch n-Pentan, i-Pentan, i-Penten, 2-Penten oder Methylbutene enthalten, wobei die Summe aller Komponenten 100 Gew.-% ergibt.

Die Diels-Alder-Reaktion zwischen dem C5-Schnitt und MSA wird im Rahmen der vorliegenden Erfindung thermisch bei Temperaturen von 40°C bis 250°C drucklos oder unter Eigendruck des Reaktionssystems in Gegenwart von Radikal-Polymerisationsinhibitoren durchgeführt. Wenn der C5-Schnitt neben Cyclopentadien auch Dicyclopentadien enthält und dies ebenfalls zur Reaktion gebracht werden soll, kann erfindungsgemäß eine zweistufige Vorgehensweise bei der Diels-Alder-Reaktion eingesetzt werden. Dabei werden zunächst monomere Diene bei niedriger Temperatur, beispielsweise bei 40 bis 140°C, mit MSA umgesetzt. Anschließend wird die Temperatur auf 150°C bis 250°C erhöht, um Dicyclopentadien umzusetzen. Erfindungsgemäß ist es möglich, die Umsetzung diskontinuierlich in einem Reaktor durch stufenweise Erhöhung der Temperatur durchzuführen. Ebenso ist es jedoch auch möglich, die Umsetzung kontinuierlich in mehreren nacheinander geschalteten Reaktoren oder Reaktorsegmenten durchzuführen, die bei unterschiedlicher Temperatur betrieben werden. Geeignete Reaktoren für die Diels-Alder-Reaktion sind beispielsweise Rührreaktoren oder Rohr-Reaktoren.

Erfindungsgemäß ist es auch möglich, die Umsetzung des C5-Schnitts mit MSA gemäß Schritt (1) so zu führen, dass das als Dicyclopentadien vorliegende Cyclopentadien nicht umgesetzt wird. Dazu wird die Temperatur bei der Diels-Alder-Reaktion unter 140°C gehalten. Eine derartige Reaktionsführung führt dazu, dass der Anteil an 5-Norbonen-2,3-dicarbonsäureanhydrid am Produktgemisch reduziert wird.

Die C5-Kohlenwasserstoffe des C5-Schnitts, die nicht mit Maleinsäureanhydrid reagieren, können nach der Umsetzung gemäß Schritt (1) leicht abgetrennt werden, beispielsweise durch Destillation.

Bei der Umsetzung des C5-Schnitts gemäß Schritt (1) wird dabei ein Gemisch verschiedener Anhydride erhalten. Es werden Gemische aus 5-Norbonen-2,3-dicarbonsäureanhydrid (Gemisch aus endo- und exo-Verbindung), 4-Methyl-4-cyclohexen-1,2-dicarbonsäureanhydrid und 3-Methyl-4-cyclohexen-1,2-dicarbonsäureanhydrid erhalten. Dabei liegen im Gemisch beispielsweise von 0 bis 30 Gew.-%, bevorzugt 10 bis 25 Gew.-%, insbesondere 15 bis 20 Gew.-% 5-Norbonen-2,3-dicarbonsäureanhydrid, vorzugsweise 17 Gew.-%, (Gemisch aus endo- und exo-Verbindung); von 0 bis 25 Gew.-%, bevorzugt 10 bis 20 Gew.-%, insbesondere 12 bis 18 Gew.-% 4-Methyl-4-cyclohexen-1,2-dicarbonsäureanhydrid, vorzugsweise 15 Gew.-%; und von 0 bis 25 Gew.-%, bevorzugt 10 bis 20 Gew.-%, insbesondere 8 bis 12 Gew.-% 3-Methyl-4-cyclohexen-1,2-dicarbonsäureanhydrid, vorzugsweise 10 Gew.-%, vor.

Erfindungsgemäß ist es möglich, ein gemäß Schritt (1) durch Umsetzung von MSA und mindestens einem C5-Dien erhaltenes Gemisch verschiedener alkylsubstituierten Cyclohexendicarbonsäureanhydride einer Behandlung zu unterziehen. Es ist jedoch ebenso möglich, dieses Gemisch ohne weitere Behandlung direkt weiter umzusetzen. Im Rahmen der vorliegenden Erfindung kann das gemäß Schritt (1) durch Umsetzung von MSA und mindestens einem C5-Dien erhaltene Gemisch also direkt weiter umgesetzt werden oder in die Reinverbindungen aufgetrennt werden, beispielsweise durch destillative Verfahren. Es ist erfindungsgemäß möglich, die nachfolgenden Reaktionen mit den Reinverbindungen durchzuführen. Ebenso ist es jedoch möglich, das bei der Umsetzung gemäß Schritt (1) erhaltene Gemisch weiter umzusetzen.

Sofern im Folgenden von Cyclohexen- oder Cyclohexandicarbonsäurederivaten die Rede ist, beziehen sich die Ausführungen sowohl auf die alkylsubstituierten als auch die unsubstituierten Dicarbonsäurederivate bzw. auf die im Schritt (1) erhaltenen Gemische.

Gemäß der Erfindung kann das in Schritt (1) erhaltene Cyclohexendicarbonsäureanhydrid anschließend aufgereinigt werden oder direkt als Rohlösung umgesetzt werden.

Die so erhaltene Roh-Lösung von Cyclohexendicarbonsäureanhydrid kann im Rahmen der vorliegenden Erfindung im Schritt (2) mittels Esterbildung zum Cyclohexendicarbonsäureester umgesetzt werden. Dabei kann die Esterbildung durch Veresterung mit einem Alkohol mit einem Alkyl-, Cycloalkyl- oder Alkoxyalkylrest oder einem Gemisch aus zwei oder mehr davon erfolgen, wobei die Alkyl-, Cycloalkyl- sowie Alkoxyalkylgruppen in der Regel 1 bis 30, vorzugsweise 2 bis 20 und besonders bevorzugt 3 bis 18 Kohlenstoffatome umfassen und verzweigt oder linear sein können. Insbesondere wird die Esterbildung mit linearen oder verzweigten, gesättigten Alkoholen mit 1 bis 20 Kohlenstoff-Atomen oder einem Gemisch aus zwei derartigen Alkoholen durchgeführt. Die Reaktion wird in dem Fachmann bekannter Weise durchgeführt, wie beispielsweise im Organikum, 18. Auflage, 1990, Deutscher Verlag der Wissenschaften Berlin, beschrieben.

Erfindungsgemäß eignen sich für die Veresterung insbesondere lineare und verzweigte Alkohole mit 1 bis 18 C-Atomen, bevorzugt 4 bis 13 C-Atomen, besonders bevorzugt 8 bis 10 C-Atomen. Als Alkohole eignen sich beispielsweise Methanol, Ethanol, lineare oder verzweigte Propanole, lineare oder verzweigte Butanole, lineare oder verzweigte Pentanole, lineare oder verzweigte Hexanole, lineare oder verzweigte Heptanole, lineare oder verzweigte Octanole, lineare oder verzweigte Nonanole, lineare oder verzweigte Decanole, lineare oder verzweigte Undecanole, lineare oder verzweigte Dodecanole, lineare oder verzweigte Tridecanole, lineare oder verzweigte Tetradecanole, lineare oder verzweigte Pentadecanole, lineare oder verzweigte Hexadecanole, lineare oder verzweigte Heptadecanole, lineare oder verzweigte Octadecanole sowie Gemische dieser Alkohole.

Bei den Alkoholen kann es sich beispielsweise handeln um Oxoalkohole, Hydroformylierungsprodukte aus C5- bis C12-Alkenen und Hydroformylierungsprodukte aus Dimer-, Trimer- und Oligomer-Ethen, aus Dimer-, Trimer- und Oligomer-Propen, aus Dimer-, Trimer- und Oligomer-n- bzw. -i-Buten, aus Dimer-, Trimer- und Oligomer-n- bzw. -i-Penten und aus Dimer-, Trimer- und Oligomer-n-bzw. -i-Hexen.

Besonders geeignet sind beispielsweise Hexanol-Gemische mit der Chemical Abstracts-Nummer (im folgenden CAS-Nr.) 68526-79-4, Heptanol-Gemische mit der CAS-Nr. 51774-11-9, Octanol-Gemische mit der CAS-Nr. 68526-83-0, Octanol-Gemische mit der CAS-Nr. 91994-92-2, 2-Ethylhexanol, Nonanol-Gemische mit der CAS-Nr. 68526-84-1, Nonanol-Gemische mit der CAS-Nr. 3452-97-9, Nonanol Gemische mit der CAS-Nr. 27458-94-2, Decanol-Gemische mit der CAS-Nr. 93821-11-5, Decanol-Gemische mit der CAS-Nr. 25339-17-7, 2-Propylheptanol, Undecanol-Gemische mit der CAS-Nr. 90604-37-8, Dodecanol-Gemische mit der CAS-Nr. 90604-37-8, Tridecanol-Gemische mit der CAS-Nr. 27458-92-0 und Tridecanol-Gemische mit der CAS-Nr. 68526-86-3.

In einer alternativen Ausführungsform der Erfindung kann die Esterbildung gemäß Schritt (2) durch Dimerisierung von Dienen am Cyclohexendicarbonsäureanhydrid selbst erfolgen. Dabei werden Ester mit C8-Seitenketten erhalten. Dadurch kann Butadien aus der vorherigen Oxidation von Raffinat auch für diesen Teil des Verfahrens sinnvoll genutzt werden. Geeignete Reaktionsbedingungen zum Aufbau der Ester-Seitenkette über Butadien-Dimerisierung sind in der FR 15 79 244 und in der JP 50-005737 beschrieben.

Ebenso ist es im Rahmen der Erfindung möglich, dass die Esterbildung gemäß Schritt (2) durch Addition von Dienen am Cyclohexendicarbonsäureanhydrid selbst oder an die entsprechende Carbonsäure erfolgt. Dieses Verfahren ermöglicht insbesondere die Herstellung von Estern mit C4-Seitenketten.

Die Hydrierung gemäß Schritt (3) des Cyclohexen-Kerns kann hier vorteilhaft gemeinsam in einem Reaktionsschritt mit der Hydrierung der ungesättigten Seitenkette erfolgen. Als Produkt der Umsetzung erhält man den entsprechenden Cyclohexandialkylester.

Die Hydrierung gemäß Schritt (3) erfolgt bevorzugt in Gegenwart eines Katalysators mit einem wasserstoffhaltigen Gas. Geeignete Katalysatoren sind beispielsweise solche von Palladium oder Platin, die an porösen Trägermaterialien gebunden sind. Weitere Reaktionsbedingungen für die Hydrierung gemäß Schritt (3) sind beispielsweise im Organikum, 18. Auflage, 1990, Deutscher Verlag der Wissenschaften Berlin, beschrieben. Für die erfindungsgemäße Hydrierung können Festbettkatalysatoren, Suspensionskatalysatoren und auch homogene Hydrierkatalysatoren eingesetzt werden, wie beispielsweise in Houben-Weyl, "Methoden der organischen Chemie", Band 4/1c beschrieben. Die Hydrierung kann je nach verwendetem Katalysator unter Normaldruck oder bei erhöhtem Druck bei Temperaturen von 20°C bis 250°C erfolgen.

Im Rahmen der Erfindung sind für die Hydrierung insbesondere auch solche Katalysatoren geeignet, wie sie in der WO 99/32427 und der DE-A 199 27 978.0 erwähnt werden.

Im Rahmen der Erfindung kann im Anschluss an Schritt (1) gemäß einer weiteren bevorzugten Ausführungsform auch zuerst eine Hydrierung des Cyclohexendicarbonsäureanhydrids zu Cyclohexandicarbonsäureanhydrid gemäß Schritt (3) erfolgen. Die Esterbildung gemäß Schritt (2) wie oben beschrieben ergibt dann erfindungsgemäß den Cyclohexandicarbonsäureester.

Mit dem erfindungsgemäßen Verfahren ist es zum einen möglich, Reinverbindungen herzustellen, zum anderen können auch Gemische verschiedener alkylsubstituierter Cyclohexandicarbonsäuren oder Derivaten davon hergestellt werden. Insbesondere ist es bei Verwendung eines Gemischs aus mehreren C5-Dienen für die Umsetzung gemäß Schritt (1) möglich, durch einen Trennschritt an beliebiger Stelle im Herstellungsverfahren, beispielsweise Destillation, die Reinverbindungen herzustellen. Ebenso ist es jedoch auch möglich, das Verfahren ohne einen Trennschritt durchzuführen und so Gemische aus mehreren alkylsubstituierten Cyclohexandicarbonsäuren oder Derivaten davon zu erhalten.

Insbesondere ist es mit dem erfindungsgemäßen Verfahren möglich, Norbonan-2,3-dicarbonsäure, 4-Methylcyclohexan-1,2-dicarbonsäure und 3-Methylcyclohexan-1,2-dicarbonsäure oder Derivate davon als Reinverbindungen, das heißt mit einem Gehalt von >90 Gew.-%, herzustellen. Ebenso können jedoch Gemische enthaltend 0 bis 70 Gew.-%, bevorzugt 10 bis 65 Gew.-%, insbesondere 15 bis 60 Gew.-%, besonders bevorzugt 20 bis 55 Gew.-% Norbanan-2,3-dicarbonsäure oder ein Derivat davon; 0 bis 70 Gew.-%, bevorzugt 10 bis 65 Gew.-%, insbesondere 15 bis 60 Gew.-%, besonders bevorzugt 20 bis 55 Gew.-% 4-Methylcyclohexan-1,2-dicarbonsäure oder ein Derivat davon und 0 bis 70 Gew.-%, bevorzugt 10 bis 65 Gew.-%, insbesondere 15 bis 60 Gew.-%, besonders bevorzugt 20 bis 55 Gew.-% 3-Methylcyclohexan-1,2-dicarbonsäure oder Derivate davon mit dem erfindungsgemäßen Verfahren hergestellt werden, wobei die Summe der einzelnen Bestandteile des Gemisches 100 Gew.-% ergibt.

Darüber hinaus betrifft die vorliegende Erfindung auch ein Gemisch, enthaltend mindestens eine alkylsubstituierte oder unsubstituierte Cyclohexandicarbonsäure oder deren Mono- oder Diester, erhältlich nach dem erfindungsgemäßen Verfahren, wobei das Gemisch mindestens Norbonan-2,3-dicarbonsäure, 4-Methylcyclohexan-1,2-dicarbonsäure und 3-Methylcyclohexan-1,2-dicarbonsäure oder einen Mono oder Diester dieser Carbonsäuren enthält.
Derartige Gemische haben beispielsweise besonders vorteilhafte Eigenschaften für die Verwendung als Weichmacher.

Besonders geeignet sind dabei die Isononylester oder 2-Ethylhexylester der jeweiligen Cyclohexandicarbonsäure, wobei das zur Herstellung des Esters verwendete Isononanol die CAS-Nummer 27458-94-2 hat. Daher betrifft die vorliegende Erfindung in einer weiteren Ausführungsform Gemische enthaltend 0 bis 70 Gew.-% Norbonan-2,3-dicarbonsäure-bis-(2-ethylhexyl)ester, 2 bis 70 Gew.-% 4-Methylcyclohexan-1,2-dicarbonsäure-bis-(2-ethylhexyl)ester und 2 bis 70 Gew.-% 3-Methylcyclohexan-1,2-dicarbonsäure-bis-(2-ethylhexyl)ester, sowie Gemische enthaltend 0 bis 70 Gew.-% Norbonan-2,3-dicarbonsäure-bis-(isononyl)ester, 2 bis 70 Gew.-% 4-Methylcyclohexan-1,2-dicarbonsäure-bis-(isononyl)ester und 2 bis 70 Gew.-% 3-Methylcyclohexan-1,2-dicarbonsäure-bis-(isononyl)ester.

Die Erfindung betrifft darüber hinaus die Verwendung eines erfindungsgemäßen Gemischs als Weichmacher für Kunststoffe, wobei der Kunststoff Polyvinylchlorid (PVC) oder Polyvinylbutyral (PVB) ist.

Das erfindungsgemäß hergestellte Gemisch wird den Kunststoffen zu Anteilen von 1 bis 80 Gew.-%, bevorzugt 5 bis 55 Gew.-%, besonders bevorzugt von 10 bis 50 Gew.-% und insbesondere von 15 bis 45 Gew.-% zugesetzt.

Im Rahmen der vorliegenden Erfindung wird unter einem Weichmacher eine Substanz verstanden, die bei Zugabe die Härte des Kunststoffs herabsetzt.

Die erfindungsgemäßen Ester besitzen eine niedrige Dichte und Viskosität. Die niedrige Dichte führt zu günstigen Volumenkosten der mit den erfindungsgemäßen Weichmachern hergestellten Kunststoffe. Die niedrige Viskosität verbessert das Verarbeitungsverhalten bei der Herstellung von Dry-blends und führt zu erniedrigten initialen Plastisolviskositäten bei der Herstellung von Pasten.

Darüber hinaus bewirken die erfindungsgemäß hergestellten Weichmacher gegenüber herkömmlichen Weichmachern eine Verbesserung der kälteelastischen Eigenschaften der mit diesen Weichmachern hergestellten Kunststoffe und eine erhöhte Thermostabilität. Die Charakterisierung der kälteelastischen Eigenschaften erfolgt vorzugsweise mit Hilfe der sogenannten Kältebruchtemperatur. Darunter wird die Temperatur verstanden, bei der ein Kunststoff in der Kälte bei mechanischer Belastung erste optisch sichtbare Beschädigungen aufweist. Die Bestimmung der Kältebruchtemperatur erfolgt nach DIN 53372. Die Charakterisierung der thermischen Stabilität erfolgt beispielsweise bei PVC mit Hilfe der sogenannten HCl-Reststabilität. Darunter versteht man die Zeitspanne, innerhalb der das weichgemachte PVC bei einer Temperatur von 200°C noch keine Zersetzung unter Abspaltung von HCl zeigt. Die Bestimmung der HCl-Reststabilität erfolgt nach der VDE-Norm 0472, §614.

Die erfindungsgemäß hergestellten Gemische können auch als Weichmacher für Gemische von verschiedenen Kunststoffen, beispielsweise von Polyvinylchlorid oder Polyvinylbutyral mit weiteren Kunststoffen ausgewählt aus der Gruppe bestehend aus Homo- und Copolymeren auf Basis von Ethylen, Propylen, Butadien, Vinylacetat, Glycidylacrylat,

Glycidylmethacrylat, Acrylaten und Methacrylaten mit Alkoholkomponenten von verzweigten oder unverzweigten C1- bis C10-Alkoholen, Styrol oder Acrylnitril eingesetzt werden.

Die mit den erfindungsgemäß hergestellten Gemisch weichgemachten Kunststoffe können beispielsweise in Gehäusen von Elektrogeräten, wie zum Beispiel Küchengeräten oder Computern, in Rohrleitungen, Apparaten, Kabeln, Draht-Ummantelungen, Fensterprofilen, im Innenausbau, im Fahrzeug- und Möbelbau, in Bodenbelägen, zur Herstellung von Dichtungen, Folien, Verbundfolien, Folien für Verbundsicherheitsglas, insbesondere PVB-Folien vom Typ TROSIFOL der Firma HT-Troplast, Schallplatten, Kunstleder, Spielzeug, Verpackungsbehältern, Klebebandfolien, Bekleidung, Beschichtungen, als Fasern für Gewebe eingesetzt werden.

Im Folgenden soll die vorliegende Erfindung anhand von Beispielen näher erläutert werden.

### BEISPIELE

### Beispiel 1: Diels-Alder Reaktion eines C5-Schnitts mit Maleinsäureanhydrid

Eine Mischung von 98 g Maleinsäureanhydrid (1,0 mol) und 180 g C5-Schnitt mit einem Gehalt von 17 Gew.-% Cyclopentadien (vorliegend als Gemisch aus etwa 2 Gew.-% Cyclopentadien und etwa 15 Gew.-% Dicyclopentadien), etwa 15 Gew.-% Isopren und etwa 10 Gew.-% Piperylen wurde mit 1000 ppm Phenothiazin als Radikalinhibitor im Autoklaven zunächst 3 h auf 70°C erhitzt und dann noch 5h auf 180°C. Anschließend destillierte man die nicht umgesetzten Kohlenwasserstoffe zunächst bei Normaldruck, anschließend im Vakuum ab. Es wurde ein Gemisch der folgenden Dicarbonsäureanhydride erhalten:
etwa 35 % 5-Norbonen-2,3-dicarbonsäureanhydrid (Gemisch aus endo- und exo-Verbindung), etwa 39% 4-Methyl-4-cyclohexen-1,2-dicarbonsäureanhydrid und etwa 26% 3-Methyl-4-cyclohexen-1,2-dicarbonsäureanhydrid.

### Beispiel 2: Hydrierung

110 g des Reaktionsgemischs aus Beispiel 1 wurden in 400 ml THF gelöst. Anschließend wurden 300 mg Palladium auf Aktivkohle (10%Pd) zugegeben und bei Raumtemperatur bei 100 bar Wasserstoffdruck hydriert. Der Katalysator wurde dann abfiltriert und das Lösungsmittel am Rotationsverdampfer abdestilliert. Man erhielt 112 g eines Gemischs aus etwa 35% 5-Norbonan-2,3-dicarbonsäureanhydrid, etwa 39% 4-Methyl-4-cyclohexan-1,2-dicarbonsäureanhydrid und etwa 26% 3-Methyl-4-cyclohexan-1,2-dicarbonsäureanhydrid.

### Beispiel 3: Veresterung

106 g des Reaktionsgemischs aus Beispiel 2 wurden mit 250 g 2-Ethylhexanol und 0,16 g Titantetrabutylat als Katalysator am Wasserabscheider gekocht, bis kein Wasser mehr gebildet wurde. Dann wurde der Katalysator mit 0,5-%iger Sodalösung hydrolysiert, die Wasserphase abgetrennt und die organische Phase nochmals mit Wasser gewaschen. Schließlich wurde das überschüssige Ethylhexanol abdestilliert, wobei die letzten Alkohol-Reste durch Strippen mit Dampf entfernt wurden. Man erhielt 166g eines Gemischs aus etwa 35% 5-Norbonan-2,3-dicarbonsäure-bis-(2-ethylhexyl)ester, etwa 39% 4-Methyl-4-cyclohexan-1,2-dicarbonsäure-bis-(2-ethylhexyl)ester und etwa 26% 3-Methyl-4-cyclohexan-1,2-dicarbonsäure- bis-(2-ethylhexyl)ester.

### Beispiel 4 und 5:

Analog zu Beispiel 3 wurde die Veresterung mit Isononanol und 2-Propylheptanol durchgeführt. Es wurden entsprechende Estergemische erhalten.

## Patentansprüche

1. Verfahren zur Herstellung einer Cyclohexandicarbonsäure, deren Mono- oder Diester umfassend die folgenden Abfolgen der Schritte (1) bis (3):
(1) Umsetzung eines Butadien-Maleinsäureanhydrid-Gemischs zu Cyclohexendicarbonsäureanhydrid in kondensierter Phase;
(2) Esterbildung aus einem Cyclohexendicarbonsäureanhydrid;
(3) Hydrierung des Cyclohexenderivats aus Schritt (2) zu dem entsprechenden Derivat von Cyclohexan;
oder
(1) Umsetzung eines Butadien-Maleinsäureanhydrid-Gemischs zu Cyclohexendicarbonsäureanhydrid in kondensierter Phase;
(3) Hydrierung des Cyclohexendicarbonsäureanhydrid zu Cyclohexandicarbonsäureanhydrid;
(2) Esterbildung aus dem Cyclohexandicarbonsäureanhydrid,
**dadurch gekennzeichnet, dass** das Butadien-Maleinsäureanhydrid-Gemisch für die Umsetzung gemäß Schritt (1) durch ein Verfahren erhalten wird umfassend die Oxidation von Strömen enthaltend n-Buten oder Butadien oder ein Gemisch davon oder durch ein Verfahren umfassend die Oxidation von n-Buten zu Butadien oder durch ein Verfahren umfassend die Oxidation von n-Buten zu einem Butadien-Maleinsäureanhydrid-Gemisch.

2. Verfahren zur Herstellung einer Cyclohexandicarbonsäure, deren Mono- oder Diester nach Anspruch 1, **dadurch gekennzeichnet, dass** das n-Buten zur Herstellung des Butadien-Maleinsäureanhydrid-Gemischs für die Umsetzung gemäß Schritt (1) durch ein Verfahren erhalten wird umfassend die Oxidation von Strömen enthaltend n-Butan.

3. Verfahren zur Herstellung einer alkylsubstituierten Cyclohexandicarbonsäure, deren Mono- oder Diester umfassend die folgenden Abfolgen der Schritte (1) bis (3):
(1) Umsetzung eines Gemischs aus Maleinsäureanhydrid und mindestens ein C5-Dien zu einem alkylsubstituierten Cyclohexendicarbonsäureanhydrid in kondensierter Phase;
(2) Esterbildung aus einem alkylsubstituierten Cyclohexendicarbonsäureanhydrid;
(3) Hydrierung des alkylsubstituierten Cyclohexenderivats aus Schritt (2) zu dem entsprechenden Derivat von Cyclohexan;
oder
(1) Umsetzung eines Gemischs aus Maleinsäureanhydrid und mindestens ein C5-Dien zu einem alkylsubstituierten Cyclohexendicarbonsäureanhydrid in kondensierter Phase;
(3) Hydrierung des alkylsubstituierten Cyclohexendicarbonsäureanhydrid zu Cyclo-hexandicarbonsäureanhydrid;
(2) Esterbildung aus dem alkylsubstituierten Cyclohexandicarbonsäureanhydrid.

4. Verfahren nach Anspruch 3, **dadurch gekennzeichnet, dass** als C5-Dien C5-Schnitte aus einem Crack-Verfahren eingesetzt werden.

5. Gemisch, enthaltend mindestens eine alkylsubstituierte oder unsubstituierte Cyclohexandicarbonsäure oder deren Mono- oder Diester, erhältlich nach einem Verfahren gemäß einem der Ansprüche 1 bis 4, wobei das Gemisch mindestens Norbonan-2,3-dicarbonsäure, 4-Methylcyclohexan-1,2-dicarbonsäure und 3-Methylcyclohexan-1,2-dicarbonsäure oder einen Mono- oder Diester dieser Carbonsäuren enthält.

6. Verwendung eines Gemischs nach Anspruch 5 als Weichmacher für Kunststoffe, wobei der Kunststoff Polyvinylchlorid oder Polyvinylbutyral ist.

## Claims

1. A process for the production of a cyclohexanedicarboxylic acid or mono- or diester thereof, comprising the following sequences of the steps (1) to (3):
(1) reaction of a butadiene-maleic anhydride mixture to give cyclohexenedicarboxylic anhydride in a condensed phase;
(2) formation of ester from a cyclohexenedicarboxylic anhydride;
(3) hydrogenation of the cyclohexene derivative from step (2) to get the corresponding derivative of cyclohexane;
or
(1) reaction of a butadiene-maleic-anhydride mixture to give cyclohexenedicarboxylic anhydride in a condensed phase;
(3) hydrogenation of the cyclohexenedicarboxylic anhydride to give cyclohexanedicarboxylic anhydride;
(2) formation of ester from the cyclohexanedicarboxylic anhydride,
wherein the butadiene-maleic-anhydride mixture for the reaction of step (1) is obtained via a process comprising the oxidation of streams comprising n-butene or butadiene or a mixture thereof or via a process comprising the oxidation of n-butene to give butadiene or via a process comprising the oxidation of n-butene to give a butadiene-maleic-anhydride mixture.

2. The process for the production of a cyclohexanedicarboxylic acid or mono- or diester thereof according to claim 1, wherein the n-butene for the production of the butadiene-maleic-anhydride mixture for the reaction of step (1) is obtained via a process comprising the oxidation of streams comprising n-butane.

3. A process for the production of an alkyl-substituted cyclohexanedicarboxylic acid, or mono- or diester thereof, comprising the following sequences of the steps (1) to (3):
(1) reaction of a mixture of maleic anhydride and at least one C5-diene to give an alkyl-substituted cyclohexenedicarboxylic anhydride in a condensed phase;
(2) formation of ester from an alkyl-substituted cyclohexenedicarboxylic anhydride;
(3) hydrogenation of the alkyl-substituted cyclohexene derivative from step (2) to get the corresponding derivative of cyclohexane;
or
(1) reaction of a mixture of maleic anhydride and at least one C5-diene to give an alkyl-substituted cyclohexenedicarboxylic anhydride in a condensed phase;
(3) hydrogenation of the alkyl-substituted cyclohexenedicarboxylic anhydride to give cyclohexanedicarboxylic anhydride;
(2) formation of ester from the alkyl-substituted cyclohexanedicarboxylic anhydride.

4. The process according to claim 3, wherein C5-cuts from a cracking process are used as C5-diene.

5. A mixture comprising at least one alkyl-substituted or unsubstituted cyclohexanedicarboxylic acid or mono- or diester thereof, obtainable by a process according to any of claims 1 to 4, where the mixture comprises at least norbonane-2,3-dicarboxylic acid, 4-methylcyclohexane-1,2-dicarboxylic acid and 3-methylcyclohexane-1,2-dicarboxylic acid or a mono- or diester of these carboxylic acids.

6. The use of a mixture according to claim 5 as plasticizer for plastics, where the plastic is polyvinyl chloride or polyvinyl butyral.

## Revendications

1. Procédé de fabrication d'un acide cyclohexane-dicarboxylique, ses mono- ou diesters, comprenant la succession suivante des étapes (1) à (3) :
(1) la mise en réaction d'un mélange de butadiène-anhydride de l'acide maléique pour former de l'anhydride de l'acide cyclohexène-dicarboxylique dans la phase condensée ;
(2) la formation d'esters à partir d'un anhydride de l'acide cyclohexène-dicarboxylique ;
(3) l'hydrogénation du dérivé de cyclohexène de l'étape (2) en le dérivé de cyclohexane correspondant ; ou
(1) la mise en réaction d'un mélange de butadiène-anhydride de l'acide maléique pour former de l'anhydride de l'acide cyclohexène-dicarboxylique dans la phase condensée ;
(3) l'hydrogénation de l'anhydride de l'acide cyclohexène-dicarboxylique en l'anhydride de l'acide cyclohexane-dicarboxylique ;
(2) la formation d'esters à partir de l'anhydride de l'acide cyclohexane-dicarboxylique,
**caractérisé en ce que** le mélange de butadiène-anhydride de l'acide maléique pour la réaction selon l'étape (1) est obtenu par un procédé comprenant l'oxydation de courants contenant du n-butène ou du butadiène ou un mélange de ceux-ci ou par un procédé comprenant l'oxydation de n-butène en butadiène ou par un procédé comprenant l'oxydation de n-butène en un mélange de butadiène-anhydride de l'acide maléique.

2. Procédé de fabrication d'un acide cyclohexane-dicarboxylique, ses mono- ou diesters selon la revendication 1, **caractérisé en ce que** le n-butène pour la fabrication du mélange de butadiène-anhydride de l'acide maléique pour la réaction selon l'étape (1) est obtenu par un procédé comprenant l'oxydation de courants contenant du n-butane.

3. Procédé de fabrication d'un acide cyclohexane-dicarboxylique à substitution alkyle, ses mono- ou diesters, comprenant la succession suivante des étapes (1) à (3) :
(1) la mise en réaction d'un mélange d'anhydride de l'acide maléique et d'au moins un diène en C5 pour former un anhydride de l'acide cyclohexène-dicarboxylique à substitution alkyle dans la phase condensée ;
(2) la formation d'esters à partir d'un anhydride de l'acide cyclohexène-dicarboxylique à substitution alkyle ;
(3) l'hydrogénation du dérivé de cyclohexène à substitution alkyle de l'étape (2) en le dérivé de cyclohexane correspondant ;
ou
(1) la mise en réaction d'un mélange d'anhydride de l'acide maléique et d'au moins un diène en C5 pour former un anhydride de l'acide cyclohexène-dicarboxylique à substitution alkyle dans la phase condensée ;
(3) l'hydrogénation de l'anhydride de l'acide cyclohexène-dicarboxylique à substitution alkyle en l'anhydride de l'acide cyclohexane-dicarboxylique ;
(2) la formation d'esters à partir de l'anhydride de l'acide cyclohexane-dicarboxylique à substitution alkyle.

4. Procédé selon la revendication 3, **caractérisé en ce que** des coupes en C5 issues d'un procédé de craquage sont utilisées en tant que diène en C5.

5. Mélange, contenant au moins un acide cyclohexane-dicarboxylique à substitution alkyle ou non substitué ou ses mono- ou diesters, pouvant être obtenu par un procédé selon l'une quelconque des revendications 1 à 4, le mélange contenant au moins de l'acide norbornane-2,3-dicarboxylique, de l'acide 4-méthylcyclohexane-1,2-dicarboxylique et de l'acide 3-méthylcyclohexane-1,2-dicarboxylique ou un mono- ou diester de ces acides carboxyliques.

6. Utilisation d'un mélange selon la revendication 5 en tant que plastifiant pour plastiques, le plastique étant le polychlorure de vinyle ou le polyvinylbutyral.
